# FASCICULE DE BREVET EUROPEEN

(11) **EP 2 780 693 B1**
(45) Date de publication et mention de la délivrance du brevet: **25.03.2020**
(21) Numéro de dépôt: 12794369.4
(22) Date de dépôt: 25.10.2012
(51) Int. Cl.: G01N 21/55, G01N 33/543, C12Q 1/68

(54) **PUCE MICROSTRUCTURÉE COMPRENANT DES SURFACES CONVEXES POUR ANALYSE PAR RÉSONANCE DES PLASMONS DE SURFACE, DISPOSITIF D'ANALYSE CONTENANT LADITE PUCE MICROSTRUCTURÉE ET UTILISATION DUDIT DISPOSITIF**
MIKROSTRUKTURIERTE CHIP MIT KONVEXEN OBERFLÄCHEN FÜR OBERFLÄCHENPLASMONRESONANZANALYSE, ANALYSEVORRICHTUNG MIT DIESEM MIKROSTRUKTURIERTEN CHIP UND VERWENDUNG DER VORRICHTUNG
MICROSTRUCTURED CHIP COMPRISING CONVEX SURFACES FOR SURFACE PLASMON RESONANCE ANALYSIS, ANALYSIS DEVICE CONTAINING SAID MICROSTRUCTURED CHIP AND USE OF SAID DEVICE

(30) Priorité: 26.10.2011 FR 1159720
(43) Date de publication de la demande: 24.09.2014
(73) Titulaire: Aryballe Technologies, 38040 Grenoble (FR)
(72) Inventeur: MERCEY, Thibaut, 75011 Paris (FR)
(74) Mandataire: Plasseraud IP
(86) Numéro de dépôt international: PCT/FR2012/052452
(87) Numéro de publication internationale: WO 2013/060989

(56) Documents cités:
- EP-A1- 2 264 438
- WO-A2-2009/021964
- FR-A1- 2 860 872
- JP-A- 2003 121 349
- JP-A- 2005 337 771
- US-A1- 2005 213 868
- US-A1- 2010 227 769
- GIUDICATTI S ET AL: "Plasmonic resonances in nanostructured gold/polymer surfaces by colloidal lithography", PHYSICA STATUS SOLIDI. A: APPLIED RESEARCH, WILEY - VCH VERLAG, BERLIN, DE, vol. 207, 1 avril 2010 (2010-04-01), pages 935-942, XP002665166, ISSN: 0031-8957, DOI: 10.1002/PSSA.200925579 [extrait le 2010-03-01]
- VALSESIA A: "Fabrication of nanostructured surfaces for the development ofadvanced biointerfaces", UNIVERSITA DEGLI STUDI DI PAVIA. SCIENTIFICA ACTA, PAVIA UNIVERSITY PRESS, IT, vol. 1, no. 1, 1 janvier 2007 (2007-01-01) , pages 153-157, XP002665167, ISSN: 1973-5219
- MANNELLI I ET AL: "Bioadhesive nanoareas in antifouling matrix for highly efficient affinity sensors", PROCEEDINGS OF SPIE, SPIE, US, vol. 7035, 12 août 2008 (2008-08-12), pages 70350Y-1, XP002665168, ISSN: 0277-786X, DOI: 10.1117/12.796969 [extrait le 2008-08-29]
- BURAK TURKER ET AL: "Grating coupler integrated photodiodes for plasmon resonance based sensing", LAB ON A CHIP, vol. 11, no. 2, 1 janvier 2011 (2011-01-01), pages 282-287, XP055011239, ISSN: 1473-0197, DOI: 10.1039/c0lc00081g
- COYLE S ET AL: "Localised plasmons in gold photonic nanocavities", SUMMARIES OF PAPERS PRESENTED AT THE QUANTUM ELECTRONICS AND LASER SCIENCE CONFERENCE. (QELS 2002). TECHNICAL DIGEST. CONFERENCE EDITION. LONG BEACH, CA, MAY 19 - 24, 2002; [TRENDS IN OPTICS AND PHOTONICS. (TOPS)], WASHINGTON, WA : OSA, US, 19 mai 2002 (2002-05-19), pages 257-257, XP010613027, DOI: 10.1109/QELS.2002.1031391 ISBN: 978-1-55752-708-0

## Description

### [Domaine de l'invention]

La présente invention concerne une puce microstructurée comprenant des surfaces convexes pour analyse par Résonance des Plasmons de Surface, un dispositif d'analyse comprenant ladite puce microstructurée, un procédé d'analyse et les utilisations dudit dispositif.

La Résonance des Plasmons de surface (ou SPR pour « Surface Plasmon Resonance » en anglais) est une technique optique qui permet de détecter de fines variations de propriétés physiques au voisinage immédiat d'une surface. Cette technique est surtout connue pour permettre de suivre en temps réel, et sans marqueur (de type fluorescent ou radioactif par exemple), des interactions biomoléculaires. Elle permet notamment de qualifier et quantifier des interactions entre des ligands immobilisés sur une surface et des analytes en solution dans un échantillon.

La SPR est un phénomène physique d'excitation collective des électrons d'un métal sur une interface métal-milieu diélectrique (ledit milieu diélectrique étant typiquement un milieu liquide ou un gaz). Pour une polarisation particulière de la lumière (polarisation Transverse Magnétique, ou « TM ») incidente sur cette interface (également dénommée « surface » dans la suite du texte) et pour un angle appelé « angle de résonance plasmon », il se produit un phénomène de résonance se traduisant par le couplage de l'énergie lumineuse incidente à une onde de surface (dénommée « plasmon de surface ») se propageant parallèlement à l'interface. Ce phénomène physique se traduit par une chute de l'intensité de la lumière réfléchie par la surface. Cette excitation n'a lieu que pour des angles d'incidence sur la surface au-delà de l'angle critique de réflexion totale (qui ne peut exister que lorsqu'un rayonnement passe d'un milieu plus réfringent d'indice optique n1 à un milieu moins réfringent d'indice optique n2, avec n1>n2). Dans ce cas, le plasmon de surface va venir « sonder » l'épaisseur optique à la surface du métal, du côté du milieu diélectrique, l'épaisseur optique étant définie comme le produit de l'indice de réfraction par l'épaisseur. Quand ces conditions sont réunies, on peut alors dire que la surface est sensible à l'effet plasmon.

Typiquement un rayonnement incident arrive selon un angle d'incidence donné sur une des faces latérales d'une puce (en général un prisme dans l'état de l'art, car il s'agit de la méthode de couplage de la lumière sur la surface sensible la plus simple à utiliser) dont une des faces est recouverte d'une couche métallique, ledit rayonnement incident est réfracté lorsqu'il entre dans le prisme (du fait de la différence d'indice optique entre le milieu constituant le prisme et le milieu optique précédent, en général de l'air) et vient se réfléchir sur ladite surface métallique. Cette configuration est bien connue de l'homme de l'art sous le nom de configuration de Kretschmann (E. Kretschmann, The détermination of the Optical Constants of Metals by Excitation of Surface Plasmons, Z Physik 241:313-324 (1971)). Des configurations équivalentes existent également en remplaçant le prisme par un réseau de diffraction pour coupler la lumière (configuration de Raether : H. Raether in "Surface Polaritons", Eds. Agranovich and Mills, North Holland Pubi. Comp., Amsterdam, 1982).

Le phénomène de SPR peut permettre en outre d'étudier des interactions biomoléculaires. Dans ce cas, des ligands sont préalablement immobilisés sur la surface métallique du prisme dans des zones définies. Ainsi, tout accrochage ultérieur d'autres molécules avec ces ligands va modifier localement l'épaisseur optique au niveau desdites zones définies, et va donc provoquer des variations des conditions de résonance et donc un déplacement de l'angle de résonance. Ce déplacement est, en première approximation, proportionnel à la quantité da matière biologique qui est venue interagir avec les ligands. Ainsi, des petites molécules vont provoquer un faible déplacement de l'angle d'incidence tandis que des molécules plus grosses vont induire un décalage angulaire bien plus important. C'est l'étude des variations de réflectivité optique liées au phénomène de résonance qui va permettre de détecter et mesurer des interactions biomoléculaires et leur évolution temporelle au niveau des zones définies.

En outre, d'autres méthodes optiques permettent également réaliser de tels phénomènes physiques sans marqueur (miroir résonnant, interférométrie, ondes acoustiques de surface, microbalance à quartz), mais ces techniques nécessitent des appareillages coûteux et inadaptés à des applications industrielles concrètes.

### [Problème technique]

Les systèmes actuels de SPR sont encombrants, coûteux et difficiles à mettre en œuvre et ne permettent donc pas de réaliser des analyses à de faibles coûts. En effet, la plupart des systèmes actuellement commercialisés nécessitent des stratégies de mesure compliquées et ne permettent des mesures que sur une zone bien précise (quasi-ponctuelle) de la surface. Les dispositifs qui permettent des analyses de plusieurs zones en parallèle sont très complexes et présentent des pièces mécaniques en mouvement, rendant ainsi le système encombrant et difficile à utiliser.

C'est pourquoi, depuis plusieurs années, de nombreuses recherches scientifiques sont dédiées au développement de dispositifs optiques SPR performants, économiques et faciles à mettre en œuvre.

Le document US5313264 (Ivarsson et al) décrit un dispositif SPR utilisant une interrogation dite « angulaire » dans lequel la surface à étudier est excitée avec un faisceau convergent et l'intensité du faisceau réfléchi est observée sur un détecteur. Cette technique ne permet cependant pas de réaliser l'étude de plusieurs zones en parallèle, à moins de juxtaposer plusieurs détecteurs (on parle d'analyse essentiellement « mono-point »).

Le document "Designing a curved surface SPR device, J Rooney et E.A.H.Hall, Sensors and Actuators B 114 (2006) 804-811" décrit un dispositif qui permet de détecter une interaction biomoléculaire sur un substrat sphérique courbe concave. Bien que ce document suggère de juxtaposer 8 fois ledit dispositif, les éléments de détection du dispositif sont également multipliés 8 fois, ce qui rend le dispositif final onéreux et encombrant.

Les documents US6862094 (Johansen et al) et US7576863 (Weibel et al) décrivent un dispositif SPR qui utilise non pas une interrogation angulaire mais une interrogation en longueur d'onde à l'aide d'un monochromateur ou d'une source de lumière blanche. Ces dispositifs permettent d'étudier plusieurs interactions biomoléculaires en parallèle mais comportent des pièces en mouvement, ce qui augmente les opérations de maintenance sur le système, et donc le coût global pour l'utilisateur.

Il existe également des dispositifs SPR permettant de réaliser de l'imagerie et de suivre à angle d'incidence et longueur d'onde fixés, l'évolution des interactions biomoléculaires dans des zones définies sur une puce. Les documents US 7678584 (Guédon et al), US 7576863 (Weibel et al), US 7551286 (Tani et al) présentent de tels dispositifs SPR. Bien que ces dispositifs permettent une analyse de plusieurs interactions en parallèle, ils présentent des pièces en mouvement ou ne permettent pas une analyse fine de l'interaction lorsqu'aucune pièce ne bouge.

De plus, tous les dispositifs décrits précédemment utilisent des puces qui sont onéreuses de par leur procédé de fabrication. Par ailleurs, comme la majorité des dispositifs sont des dispositifs « mono-point », le prix par point d'analyse est important.

Par ailleurs, on peut également citer dans l'état de l'art le document WO2009/021964A2 (Maccraith and al) qui décrit une plate-forme optique destinée à détecter des analytes par fluorescence. Dans ce document, la surface plane supérieure d'un réseau de protubérances de forme paraboloïde est métallisée puis fonctionnalisée par des espèces biologiques. Puis, un signal de fluorescence est excité grâce à un effet plasmonique généré par la géométrie paraboloïde des protubérances, qui permet d'obtenir un faisceau incident sur ladite surface plane métallisée au-delà de l'angle critique.

Cependant, dans ce document, aucune information ne peut être déduite directement des caractéristiques de l'onde plasmon elle-même car elle ne sert qu'à l'émission indirecte de lumière par luminescence. De plus, la surface utile de détection est une surface plane métallisée, et ne permet que de réaliser une analyse à un angle θ précis.

Enfin, il existe également dans l'état de l'art de nombreux documents décrivant des puces présentant des nanostructures à leur surface, qui pour certaines d'entre elles, utilisent le phénomène physique des plasmons de surface localisés (LSPR). A titre d'exemple, le document *"*Grating coupler integrated photodiodes for plasmon resonance based sensing, B. Turker and al., Conférence on lasers and Electro-Optics 2011*"* décrit des biopuces disposant à leur surface de nanostructures agencées selon un réseau périodique. Ce réseau de nanostructures est utilisé pour coupler une lumière incidente à l'onde plasmon de l'interface métal/diélectrique du réseau. Le document "Localised plasmons in gold photonic nanocavities, S. Coyle and al., Quantum Electronics and Laser Science Conférence 2002" présente quant à lui une surface nanostructurée avec des nanocavités d'or. Ces nanocavités d'or mettent en jeu le phénomène physique des plasmons de surface localisés (LSPR) qui diffère du phénomène SPR et qui mène à une amplification des signaux plasmoniques.

Ainsi, dans les deux documents précités, non seulement la réalisation de réseaux et de cavités à une échelle nanométrique est difficile, mais aussi il est nécessaire de faire appel à des pièces mobiles pour évaluer la variation de réflectivité de la couche métallique en fonction de l'angle d'incidence du faisceau lumineux.
Giudicatti Silvia and Al et I. Mannelli and al décrivent une puce structurée présentant une pluralité de protubérances nanométriques.
FR 2860872 décrit un micro-capteur ou un nano-capteur à plasmons de surface localisés ou délocalisés.
JP 2003 121349 décrit un capteur à résonance des plasmons de surface.

Enfin, les dispositifs les plus compacts utilisent des puces dont la surface est concave. Or, la réalisation de pièce de forme concave est difficile à mettre en œuvre et coûteuse.

Il existe donc un réel besoin en un dispositif SPR compact, ne présentant pas de pièce en mouvement, économique, et comprenant une puce simple à réaliser permettant ainsi de réaliser des analyses SPR à de faibles coûts.

### [Description de l'invention]

Le présent inventeur a trouvé qu'un dispositif d'analyse par SPR comprenant une puce microstructurée présentant une architecture particulière permettait de répondre à ces exigences.

On utilisera indifféremment le terme « puce » ou « puce microstructurée » dans la suite du texte.

On utilisera indifféremment le terme , « rayonnement » ou « faisceau » dans la suite du texte.
La figure 1 représente en coupe des protubérances selon différents modes de réalisation de l'invention.
La figure 2 représente en coupe des cavités selon différents modes de réalisation de l'invention.
La figure 3 représente une puce microstructurée selon l'invention dotée de protubérances disposées sous forme de matrice.
La figure 4 représente la face supérieure d'une puce microstructurée selon l'invention dotée de protubérances disposées sous forme de goulottes le long de l'axe X.
La figure 5 représente en coupe une protubérance sans tilt irradiée par un rayonnement incident monochromatique collimaté et polarisé.
La figure 6 représente en coupe une protubérance présentant un tilt d'un angle β, irradiée par un rayonnement incident monochromatique collimaté et polarisé.
La figure 7 représente une puce selon un mode de réalisation de l'invention qui présente des distances entre les surfaces planes séparant les protubérances et la base différentes.
La figure 8 représente un dispositif comprenant une puce microstructurée présentant des distances entre les surfaces planes séparant les protubérances et la base identiques.
La figure 9 représente un dispositif comprenant une puce microstructurée présentant des distances entre les surfaces planes séparant les protubérances et la base différentes.
La figure 10 montre une courbe plasmon représentative de différentes plages angulaires étudiées (sans tilt).
La figure 11 montre une courbe plasmon représentative de différentes plages angulaires étudiées (avec tilt).
La figure 12 représente un exemple d'image sur une caméra de 3 protubérances d'une puce microstructurée selon l'invention.
La figure 13 représente une puce selon l'invention comprenant 16 protubérances dont au moins une est différente des autres.
La figure 14 représente le pourcentage de réflectivité en fonction de l'angle d'incidence θ.
La figure 15 représente les courbes plasmons avant et après accrochage de la bactérie *Escherichia coli* sur les protubérances fonctionnalisées avec des ligands spécifiques de la bactérie.
La figure 16 représente les courbes plasmons avant et après accrochage des shigatoxines sur les protubérances fonctionnalisées avec des ligands spécifiques des shigatoxines.

### Puce microstructurée

Un premier objet de l'invention est une puce microstructurée pour analyse par résonance des plasmons de surface (SPR) se présentant sous la forme d'un solide constitué d'une base, d'une face supérieure dont au moins une partie est recouverte d'une couche métallique, et d'au moins une face latérale, dans laquelle :
- la dite face supérieure est dotée de zones d'une taille allant de 1 µm à 1000 µm destinées à recevoir des espèces à analyser choisies parmi n protubérances et m cavités; et
- lorsque n+m≥ 2, lesdites zones sont séparées les unes des autres par des surfaces planes, lesdites surfaces planes étant parallèles à un plan (XY),
   avec n allant de 1 à j et m allant de 0 à i, j et i étant des entiers ; et
- lesdites zones présentent une surface courbe de rayon de courbure R moyen situé dans un plan orthogonal au plan (XY)dans le plan (YZ) ;
- le rayon de courbure R moyen de la surface courbe est compris entre 0,1 et 600mm,
caractérisée en ce que au moins une des n protubérances et m cavités présente un rayon de courbure différent des autres.

Selon l'invention, m est différent de 0 (m>0). En d'autres termes, la face supérieure de la puce est dotée de zones de taille micrométrique destinées à recevoir des espèces à analyser choisies parmi au moins une protubérance et au moins une cavité.

Par « puce microstructurée », on entend une puce présentant des zones de taille micrométrique destinées à recevoir des espèces à analyser. Ainsi, la puce n'est pas nécessairement de taille micrométrique mais comprend des zones qui sont de taille micrométrique.

Par « zones de taille micrométrique », on entend des zones qui présentent au moins deux dimensions sur trois de taille micrométrique, lesdites dimensions micrométriques allant de 1µm à 1000µm, et préférentiellement de 10pm à 500pm.

La troisième dimension de la zone n'est donc pas nécessairement micrométrique et peut présenter des distances comprise entre 1mm et 20mm, de préférence entre 1mm et 10mm.

Les zones selon l'invention présentent des géométries particulières sous la forme de protubérances et éventuellement de cavités qui seront décrites par la suite. Ce sont au niveau de ces zones recouvertes d'une couche métallique que l'effet plasmon sera observé.

Selon l'invention, la puce comprend un nombre de zones (c'est-à-dire n protubérances et m cavités) compris entre 1 (lorsque m=0 et n=1) et 500000, de préférence entre 10 et 10000 et plus préférentiellement entre 25 et 400.

Selon l'invention, au moins une partie de la face supérieure est revêtue de la couche métallique. Elle représente la surface utile de détection (appelée également surface sensible par la suite). Selon l'invention, au moins une zone de taille micrométrique choisie parmi n protubérances et m cavités est revêtue de la couche métallique.

Selon un mode de réalisation particulier, la face supérieure est entièrement recouverte par la couche métallique.

Ainsi, selon un mode de réalisation particulier combiné au précédent, non seulement les zones mais également les surfaces planes séparant lesdites zones sont destinées à recevoir des espèces à analyser.

Pour faciliter la description qui suit, le référentiel orthonormé direct (XYZ) est utilisé dans lequel les surfaces planes séparant les zones sont parallèles au plan (XY) et dans lequel l'axe Z est orienté vers le bas.

Par « surfaces planes », on entend des surfaces planes ou sensiblement planes pouvant présenter quelques défauts.

La surface plane séparant deux zones destinées à recevoir des espèces à analyser est appelée surface inter-zones. Ainsi, on utilisera indifféremment les termes suivants : surfaces planes ou surfaces inter-zones ou encore surfaces planes inter-zones. Plus spécifiquement :
- lorsque la surface plane sépare deux zones qui sont des cavités, on parle de surface inter-cavité;
- lorsque la surface plane sépare deux zones qui sont des protubérances, on parle de surface inter-protubérance; et
- lorsque la surface plane sépare une zone qui est une cavité et une autre zone qui est une protubérance, on parle de surface inter-cavité-protubérance.

Selon un mode de réalisation, les zones sont disposées sous la forme d'une matrice sur la face supérieure de la puce. Ainsi, les surfaces planes séparent les zones à la fois selon l'axe X et l'axe Y.

Selon un autre mode de réalisation, les zones sont disposées sous forme de goulottes continues le long de la face supérieure de la puce. Ainsi, selon ce mode de réalisation, les surfaces planes séparent les zones selon l'axe X ou selon l'axe Y.

Selon un mode de réalisation, lorsque n+m≥2; les zones sont séparées d'une distance D selon l'axe Y et d'une distance D' selon l'axe X par les surfaces planes (ou surfaces inter-zones); D et D'étant compris entre 0µm (dans le cas d'une goulotte continue selon l'un des axes X ou Y) et 5mm, de préférence entre 50pm et 5mm, de préférence entre 200 et 1000 µm et de manière particulièrement préférée entre 300 et 700pm.

Selon un mode de réalisation, lorsque n+m≥2, les zones sont séparées d'une distance centre à centre CTC comprise entre 10pm et 25000pm, de préférence entre 50pm et 5000pm et plus préférentiellement entre 100µm et 1000µm.

Selon un mode de réalisation, les surfaces planes appartiennent à des plans parallèles au plan (XY); la face supérieure se présente alors sous la forme de marches d'escalier.

Selon un autre mode de réalisation, les surfaces planes appartiennent à un même plan (XY) (c'est à dire Z est constant).

Par « protubérance », on entend une excroissance sur la face supérieure de la puce, ladite excroissance se trouvant entre deux surfaces planes (appelées également surface inter-zone).

La protubérance peut être définie soit en 3 dimensions (repère XYZ) soit en 2 dimensions (coupe dans un plan).

Ainsi, on appelle protubérance, un volume dont toutes les coordonnées se trouvent au-dessus d'un plan fictif reliant les deux surfaces planes inter-zones adjacentes à ladite protubérance.

Par la suite, la protubérance sera décrite dans le plan (YZ).

Ainsi, une protubérance selon l'invention, décrite dans le plan (YZ),est définie par au moins une courbe de rayon de courbure R moyen et/ou au moins une droite.

Lorsque la protubérance est définie uniquement par une courbe, alors la courbe est nécessairement convexe (c'est-à-dire rayon de courbure selon l'axe +Z).

Lorsque la protubérance est définie par au moins une courbe et au moins une droite, alors la courbe peut être soit concave (c'est-à-dire rayon de courbure selon l'axe - Z), soit convexe.

Selon un mode de réalisation particulier, la protubérance est définie par deux droites séparées par une courbe.

Selon un mode de réalisation particulier combiné au précédent, les deux droites sont parallèles.

Selon un autre mode de réalisation pouvant être combiné au précédent, les deux droites présentent des dimensions différentes.

Par « cavité », on entend un creux dans la face supérieure de la puce, le dit creux se trouvant entre deux surfaces planes (appelées également surface inter-zones).

La cavité peut être définie soit en 3 dimensions (repère XYZ) soit en 2 dimensions (coupe dans un plan).

Ainsi, on appelle cavité, un volume dont toutes les coordonnées se trouvent sous un plan fictif reliant les deux surfaces planes inter-zones adjacentes à ladite cavité.

Par la suite, la cavité sera décrite dans le plan (YZ) .

Ainsi, une cavité selon l'invention est décrite dans le plan (YZ) et est définie par au moins une courbe de rayon de courbure R moyen et/ou au moins une droite.

Lorsque la cavité est définie uniquement par une courbe, alors la courbe est nécessairement concave (c'est-à-dire rayon de courbure selon l'axe -Z).

Lorsque la cavité est définie par au moins une courbe et au moins une droite, alors la courbe peut être soit concave, soit convexe (c'est-à-dire rayon de courbure selon l'axe +Z).

Selon un mode de réalisation particulier, la cavité est définie par deux droites séparées par une courbe.

Selon un mode de réalisation particulier combiné au précédent, les deux droites sont parallèles.

Selon un autre mode de réalisation pouvant être combiné au précédent, les deux droites présentent des dimensions différentes.

Ainsi, selon l'invention, les zones de la puce (n protubérances et m cavités), décrites dans le plan (YZ), sont définies par une courbe de rayon de courbure moyen R (concave pour une cavité et convexe pour une protubérance); le rayon de courbure R étant compris entre 0,1mm et 600mm, de préférence entre 0,3mm et 300mm.
A titre d'exemple, on peut citer n protubérances et m cavités de forme semi-sphérique, semi-elliptique ou semi-cylindrique (repère (XYZ)).

Selon un mode de réalisation préféré de l'invention, les n protubérances et m cavités présentent une forme semi-cylindrique dans le repère (XYZ).

Selon l'invention, le rayon de courbure des zones (i.e. des n protubérances et des m cavités) est perpendiculaire au plan (XY), c'est-à-dire selon l'axe Z.

Selon l'invention, la base de la puce peut être une surface plane ou courbe, ou une arête, ou un point.

Selon un mode de réalisation préféré, la base de la puce est une surface plane qui, de manière préférée, est parallèle aux surfaces planes (appelées aussi surfaces inter-zones).

Selon un autre mode de réalisation pouvant être combiné au précédent, les surfaces planes appartiennent au même plan (XY). Autrement dit, si la base est parallèle aux dites surfaces planes, les distances entre les surfaces planes et la base de la puce sont identiques. Dans ce cas, on dit que la face supérieure est parallèle à la base.

Selon un autre mode de réalisation, les surfaces planes appartiennent à de multiples plans parallèles au plan (XY). Autrement dit, si la base est parallèle aux dites surfaces planes, les distances entre les surfaces planes et la base de la puce sont différentes (face supérieure en forme de marche d'escalier).

Par « distances entre les surfaces planes (ou surfaces inter-zones) et la base », il est fait référence aux hauteurs de la puce, c'est-à-dire à la longueur de la perpendiculaire reliant les surfaces et la base ou une prolongation de la base.

Selon l'invention, la (ou les) face(s) latérale(s) de la puce peut (peuvent) être plane(s) (perpendiculaire(s) ou non à la base et/ou à la face supérieure de la puce) ou courbe(s).

Selon un mode de réalisation préféré, la (ou les) face(s) latérale(s) de la puce est (sont) plane(s).

Selon un autre mode de réalisation préféré, au moins une face latérale de la puce est perpendiculaire à la base et/ou à la face supérieure.

Selon un autre mode de réalisation, la face supérieure est parallèle à la base de la puce.

Selon un mode de réalisation particulier de l'invention, la puce est accolée à un prisme bien connu de l'état de l'art.

Ainsi, selon un mode de réalisation particulier, la puce se présente sous la forme d'un parallélépipède (c'est-à-dire une puce présentant des surfaces planes appartenant à un même plan (XY) et une base parallèle aux dites surfaces planes). Selon ce mode de réalisation, les hauteurs des surfaces latérales sont de faibles dimensions de l'ordre de 0,1mm à 20mm, de préférence de 1mm à 10mm).

Par espèces à analyser, on entend par exemple des matériaux, des gaz ou des espèces biologiques telles que de l'ADN simple ou double-brin, des protéines, des bactéries, des toxines, des virus, des mycoplasmes, des agents chimiques ou toute autre espèce biologique ou chimique susceptible d'interagir avec d'autres espèces biologiques ou chimiques.

Selon un mode de réalisation préféré, les espèces à analyser sont des espèces biologiques, telles que des bactéries pathogènes comme *Salmonella spp., Listeria monocytogenes, Clostridium difficile,* ou encore *Campylobacter spp.*

Le présent inventeur a démontré qu'il était possible en utilisant la puce selon l'invention d'étudier avantageusement des souches d'*Escherichia coli* productrices de shigatoxines (STEC), car elle permet l'analyse simultanée de grosses molécules (bactéries elles-mêmes), mais également de petites toxines qu'elles produisent.

Selon un autre mode préféré de l'invention, les n protubérances et m cavités seront fonctionnalisées avec différents anticorps monoclonaux spécifiques de biomarqueurs.

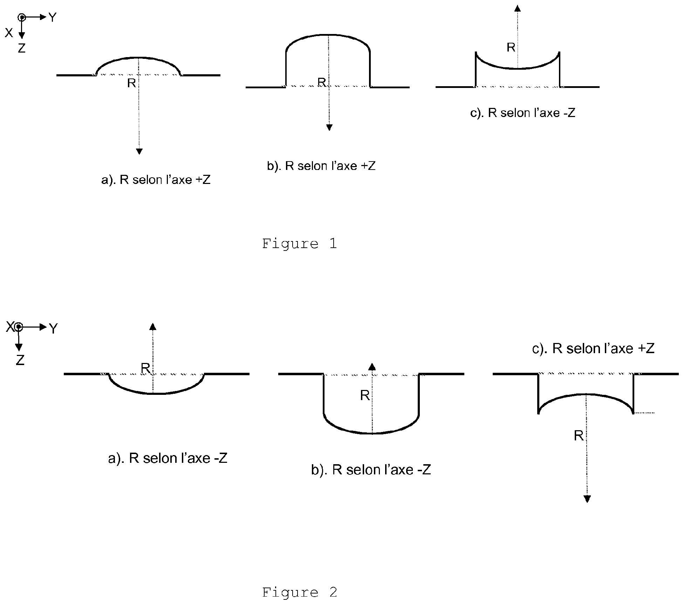

La figure 1 représente une coupe selon le plan (ZY) d'une protubérance selon différents modes de réalisation. Sur chacune de ces figures, la protubérance est représentée par une surface dans le plan (XY) (ou par un volume dans le repère XYZ) dont tous les points se situent au-dessus (Z négatif) d'une droite fictive représentée en pointillée (ou plan fictif dans le repère XYZ pour un volume) reliant les deux surfaces planes adjacentes à la protubérance :
- dans le cas a) : la protubérance est définie uniquement par une courbe de forme convexe de rayon de courbure R (c'est-à-dire présentant un rayon de courbure selon l'axe +Z);
- dans le cas b) : la protubérance est définie par deux droites parallèles séparées par une courbe de rayon de courbure R de forme convexe;
- dans le cas c) : la protubérance est définie par deux droites parallèles séparées par une courbe de rayon de courbure R de forme concave (c'est-à-dire présentant un rayon de courbure selon l'axe -Z).

La figure 2 représente une coupe selon le plan (ZY) d'une cavité selon différents modes de réalisation. Sur chacune de ces figures, la cavité est représentée par une surface dans le plan (XY) (ou par un volume dans le repère XYZ) dont tous les points se situent au-dessous (Z positif) d'une droite fictive représentée en pointillée (ou plan fictif dans le repère XYZ pour un volume) reliant les deux surfaces planes adjacentes à la cavité
- dans le cas a) : la cavité est définie uniquement par une courbe de forme concave de rayon de courbure R; (c'est-à-dire présentant un rayon de courbure selon l'axe -Z);
- dans le cas b) : la cavité est définie par deux droites parallèles séparées par une courbe de forme concave de rayon de courbure R;
- dans le cas c) : la cavité est définie par deux droites parallèles séparées une courbe de rayon de courbure R de forme convexe (c'est-à-dire présentant un rayon de courbure selon l'axe +Z).

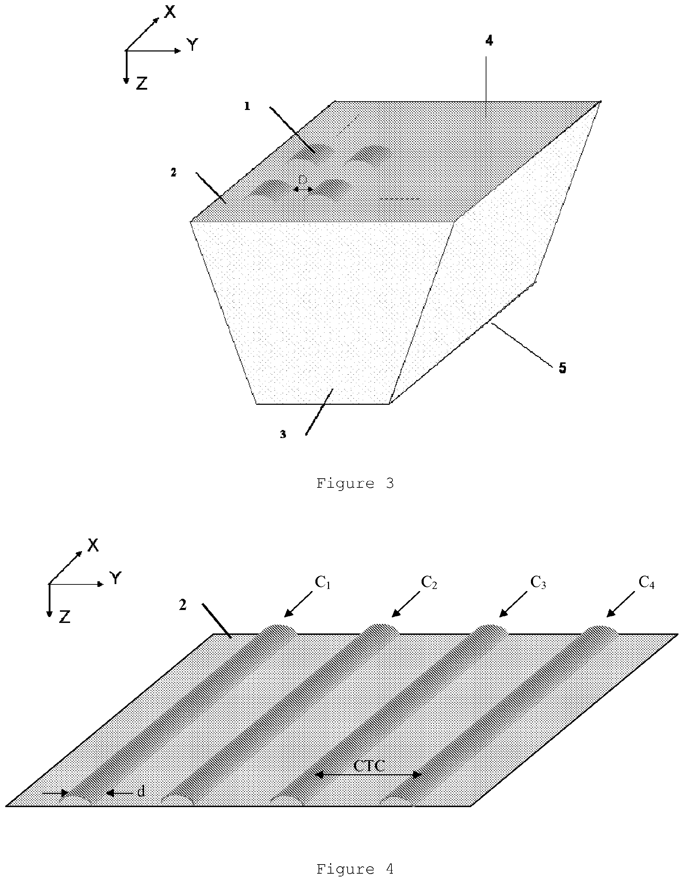

La figure 3 représente une puce microstructurée 3 dans laquelle la face supérieure 4 comprenant des surfaces planes inter-protubérances est parallèle à la base 5 de la puce 3. La face supérieure 4 est recouverte d'une couche métallique 2 et est dotée de protubérances 1 sensibles à l'effet plasmon, destinées à recevoir des espèces à analyser.

Sur la figure 3, les protubérances 1 sont séparées les unes des autres d'une distance D selon l'axe Y et d'une distance D' selon l'axe X par des surfaces planes (appelées également surfaces inter-protubérances).

La puce microstructurée 3 peut être réalisée en tout type de matériaux permettant la propagation de la lumière. On peut citer par exemple, le verre, un cristal ou des matières plastiques.

Selon un mode de réalisation préféré, pour des raisons de coût, la puce 3 est réalisée en matière(s) plastique(s) comme par exemple le PMMA (polyméthacrylate de méthyle), le PC (Polycarbonate), le PS (Polystyrène), le SU-8 (résine photosensible négative à base d'époxy) ou le PDMS (Polydiméthylsiloxane).

Selon un mode de réalisation particulier, lorsque la puce est accolée à un prisme, elle peut être réalisée dans un matériau différent de celui du prisme.

La couche métallique 2 qui recouvre la couche supérieure 4 de la puce 3 (et en particulier les protubérances 1) peut être réalisée avec divers métaux tels que l'or, l'argent, le platine ou l'aluminium.

Selon un mode de réalisation préféré, en raison de ses très bonnes propriétés anti-corrosives, la couche métallique 2 est en or.

L'épaisseur de la couche métallique 2 est comprise entre 10nm et 200nm, de préférence entre 30nm et 100 nm et plus préférentiellement encore entre 40nm et 50nm.

Selon un autre mode de réalisation combiné aux précédents, une fine couche de chrome est utilisée comme couche de pré-accrochage de l'or sur la face supérieure 4 de la puce 3.

La figure 4 représente la face supérieure d'une puce microstructurée selon l'invention entièrement recouverte d'une couche métallique 2, ladite puce microstructurée est dotée de protubérances qui sont disposées sous forme de goulottes le long de l'axe X formant ainsi 4 colonnes C₁-C₄. Dans la figure 4, d représente le diamètre de la goulotte et CTC représente la distance centre à centre entre deux goulottes successives.

La puce selon l'invention permet d'adapter la sensibilité de chaque zone (c'est-à-dire des n protubérances et m cavités) afin d'étudier des espèces biologiques très différentes.

Selon l'invention, au moins une des n protubérances et m cavités présente un rayon de courbure différent des autres.

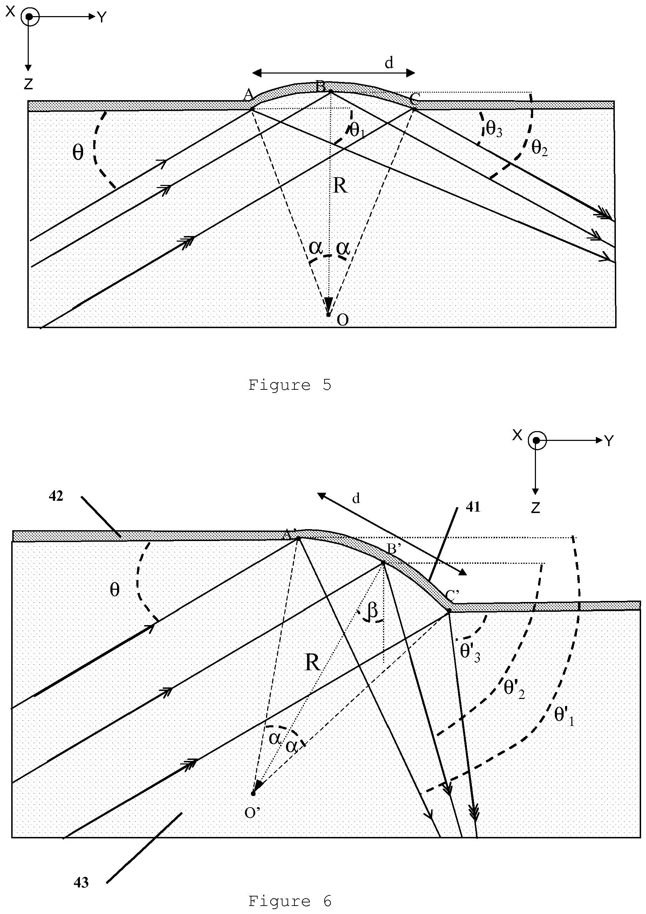

La figure 5 représente un agrandissement en coupe de la figure 3 de la protubérance de surface courbe convexe 1 de la puce 3 (recouverte de la couche métallique 2) qui est irradiée par un faisceau collimaté monochromatique d'angle d'incidence θ et polarisé linéairement suivant la polarisation TM.

La protubérance, de forme semi-cylindrique (dans le repère XYZ), présentant une surface définie par une unique courbe (dans le plan ZY) sur les figures 3 et 5, est caractérisée par son rayon de courbure R et par les deux demi-angles α (définissant ainsi un angle total de 2α).

Le rayon de courbure R et le demi-angle α vont ainsi définir la longueur de la corde d, ou encore le diamètre de ladite cavité.

Du fait de la réflexion sur la surface sensible (ou protubérance 1), l'angle d'incidence sur les deux extrémités A et C vont être respectivement, pour un angle d'incidence moyen du faisceau collimaté de θ, de θ₁=θ-2α et θ₃=θ+2α. Ainsi, le faisceau réfléchi (θ₁ pour le point A, θ₂ pour le point B, θ₃ pour le point C) par ladite protubérance 1 présente une largeur angulaire Δθ (Δθ=θ₃-θ₁) égale à 4α, centrée autour de l'angle θ_{moyen} (correspondant à l'angle θ₂ sur la figure 5). Dans ce cas, on a θ_{moyen}=θ₂=θ.

Il est important de noter que le choix du rayon de courbure R des n protubérances et m cavités de la puce selon l'invention est très important car il va déterminer, en fonction des paramètres physiques essentiels (indice optique de la puce n_{P}, indice optique du milieu diélectrique extérieur nₑ, angle d'incidence moyen du faisceau collimaté θ et la taille des espèces biologiques que l'on souhaite analyser), la sensibilité et la dynamique angulaire de la mesure, pour chacune des n protubérances et m cavités.

Par « dynamique angulaire de la mesure », on entend la plage angulaire qui va pouvoir être visualisée lors des analyses.

Par « sensibilité », on entend la plus petite variation d'épaisseur optique qu'on va pouvoir mesurer sur la surface sensible.

En effet, si le rayon de courbure est très grand, on va se rapprocher d'un plan, donc les angles θ₁ et θ₃ seront très proches (la plage angulaire d'analyse Δθ sera donc très petite) rendant cette configuration particulièrement adaptée à l'analyse de petites molécules (c'est-à-dire une bonne sensibilité). A l'opposé, un rayon de courbure très petit permettra d'observer la courbe plasmon dans son ensemble, avec certes une sensibilité de mesure plus faible mais plus adaptée à l'analyse de grosses molécules.

Il est ainsi possible selon l'invention, d'adapter la sensibilité de mesure pour différentes espèces étudiées.

Selon un autre mode de réalisation pouvant être combiné au précédent, au moins une des n protubérances et m cavités présente un tilt (ou basculement) d'un angle β.

Selon ce mode de réalisation, le rayon de courbure R est dévié par rapport à l'axe Z.

La figure 6 représente un agrandissement en coupe d'une protubérance de surface courbe convexe 41 d'une puce 43 (dont la face supérieure est recouverte d'une couche métallique 42) qui présente un tilt d'un angle β et qui est irradiée par un faisceau monochromatique collimaté avec un angle d'incidence moyen θ et polarisé linéairement suivant la direction transverse magnétique TM.

Dans le cas d'un tilt β de la surface sensible (ici de la protubérance) par rapport à la perpendiculaire au plan moyen de la surface sensible, le plan moyen étant défini comme le plan parallèle aux surfaces planes inter-protubérances, le faisceau réfléchi présentera encore une largeur angulaire Δθ égale à 4α, mais cette fois-ci centrée autour de l'angle moyen γ'_{moyen} (représenté par θ₂ sur la figure 3) tel que γ'_{moyen} =θ+2β pour une surface convexe.

Selon un mode de réalisation, l'angle β est défini de la façon suivante : 0° <β≤80°, de préférence 15°≤ β≤45°.

Il est ainsi possible selon l'invention d'ajuster, pour chacune des n protubérances et m cavités, à la fois la largeur angulaire étudiée, mais aussi l'angle moyen (c'est-à-dire θ₂ ou θ'₂ représenté sur les figures 5 et 6 selon que la protubérance soit tiltée ou pas) de cette plage angulaire, permettant ainsi d'adapter la sensibilité de mesures pour différentes espèces au sein d'une même puce, lors d'une même expérience.

Ainsi, selon un mode de réalisation particulier, au moins une des zones (c'est à dire au moins une des n protubérances et des m cavités) présente à la fois un rayon de courbure différent et une orientation différente par rapport aux autres zones de la puce.

Selon un autre mode de réalisation, pouvant être combiné aux précédents, au moins une distance entre la base et les surfaces planes (appelée également surface inter-zone) est différente des autres.

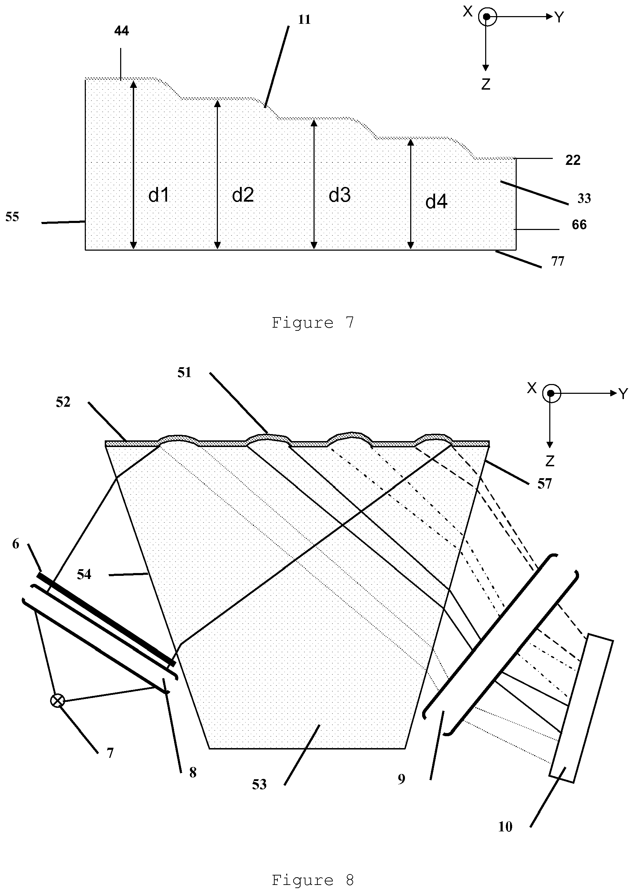

La figure 7 représente une puce 33 dont la face supérieure 44 qui est recouverte d'une couche métallique 22 est dotée de protubérances de forme courbe 11. Les faces latérales (55,66) de la puce 33 sont perpendiculaires à la base 77 et aux surfaces planes.

Les distances séparant les surfaces planes entre les protubérances 11 (ou surfaces inter-protubérances) et la base 77 sont toutes différentes les unes des autres avec d4<d3<d2<d1. Sur la figure 7, les surfaces inter-zones appartiennent à de multiples plans parallèles au plan (XY)(5 plans représentés).

La puce selon l'invention peut être réalisée par différentes méthodes qui comportent nécessairement une étape de fabrication de la puce suivie d'une étape de dépôt d'au moins une couche mince métallique.

Parmi les méthodes de fabrication, on peut citer l'injection haute pression, l'usinage mécanique direct, l'emboutissage à chaud, la gravure au plasma, la photolithographie ou l'ablation par laser.

Selon un mode de réalisation préféré, la puce est fabriquée par injection haute pression.

La réalisation de la puce selon l'invention nécessite l'utilisation d'un moule de forme concave afin de réaliser une protubérance de forme convexe ce qui permet ainsi de diminuer son coût de production. En effet, un homme de l'art sait que l'étape la plus coûteuse lors de la mise au point d'une production de composants par le procédé d'injection haute pression est la réalisation du moule (ou « master »). Le fait d'avoir une surface finale de la pièce à réaliser avec des protubérances convexes va permettre de réaliser un moule avec des formes concaves, ce qui revient à retirer de la matière dans le « master », ce qui est très simple à réaliser avec des méthodes d'usinage mécanique classique.

Parmi les méthodes de dépôt de couches minces métalliques, on peut citer la pulvérisation cathodique, des techniques d'évaporation sous vide, ou des techniques de dépôt à froid.

Les techniques de dépôt à froid sont utiles notamment dans le cas d'un support en plastique car le plastique ne supporte pas des montées en températures importantes.

### Dispositif d'analyse

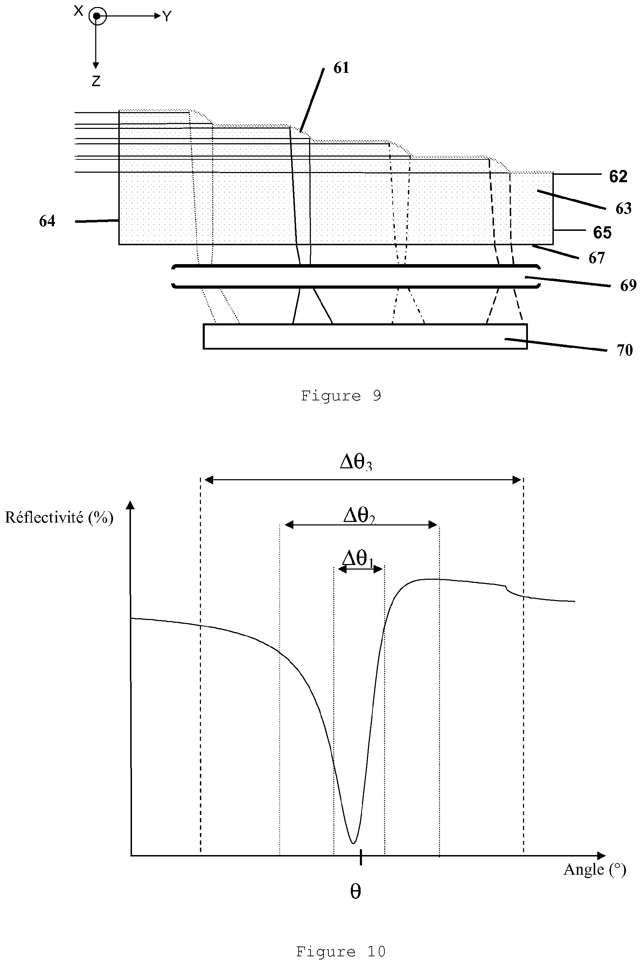

Les figures 8 et 9 représentent différents modes de réalisation de dispositifs de mesure comprenant la puce microstructurée décrite précédemment.

Ainsi, un autre objet de la présente invention est un dispositif d'analyse par SPR comprenant:
- une source lumineuse 7 destinée à générer un faisceau incident;
- éventuellement un système optique de collimation 8;
- un système polarisant 6;
- une puce microstructurée (3;33;43; 53; 63) telle que décrite précédemment disposée dans le trajet optique dudit faisceau incident;
- éventuellement un système optique d'imagerie (9; 69)
- un détecteur (10; 70).

Selon un mode de réalisation, le couplage entre l'énergie du faisceau incident et l'onde de surface de la surface métallique de la puce est réalisé par la puce elle-même.

Selon un autre mode de réalisation, le moyen de couplage est un prisme, un guide d'onde ou un réseau de diffraction.

Ainsi, selon un mode de réalisation particulier, lorsque le couplage est réalisé par un prisme, la puce selon l'invention est accolée audit prisme de telle façon que la base de la puce est mise en contact avec la face supérieure du prisme ne présentant pas de surface métallique à l'aide d'une huile de couplage d'indice qui est une méthode bien connue de l'homme de l'art.

Selon un autre mode de réalisation, la puce est accolée à un guide d'onde.

Selon encore un autre mode de réalisation particulier, la puce est accolée à un réseau de diffraction.

Selon l'invention, la source 7 peut être par exemple une lampe à vapeur de mercure, une lampe à incandescence, une diode laser, un laser, une diode électroluminescente (LED) ou une diode électroluminescente organique (OLED).

Selon un mode de réalisation préféré, la source 7 est une LED monochromatique. Par monochromatique, on entend une LED dont la largeur spectrale à mi-hauteur n'excède pas 40nm.

Selon l'invention, différentes gammes de longueur d'onde peuvent être utilisées telles que le visible ou le proche infrarouge (IR).

Selon un mode de réalisation préféré, une longueur d'onde comprise entre 790 et 825nm (proche IR) est utilisée.

Selon l'invention le faisceau peut être collimaté. Pour ce faire, différentes techniques bien connues de l'homme de l'art pourront être utilisées.

A titre d'exemple, on peut utiliser comme système optique de collimation 8 une première lentille convergente qui permet de focaliser la lumière émise par la source 7 sur un trou de diamètre Φ, ledit trou étant dans le plan focal d'une lentille convergente ce qui permet de générer le faisceau collimaté.

Selon un mode de réalisation, le système optique de collimation 8 est intégré à la source 7.

Selon l'invention, le système polarisant 6 permet de travailler en mode Transverse Magnétique (ou TM, ou polarisation-p).

A titre d'exemple, on peut citer un polariseur linéaire ou un cube séparateur polarisant.

Selon un mode de réalisation préféré, le système polarisant 6 permet de basculer facilement d'une polarisation TM à une polarisation TE (Transverse Électrique), et inversement. Afin d'éviter tout mouvement de pièce, ceci peut être réalisé à l'aide d'une lame à cristaux liquides, pilotée par des courants et tensions électriques.

Selon l'invention, le détecteur (10; 70) peut être par exemple une caméra CCD, CMOS ou être une matrice de photodétecteurs.

Selon un mode de réalisation préféré, les caméras fonctionnent sur 8, 10, 12 ou 16 bits et de préférence sur 10 ou 12 bits.

Selon un mode de réalisation, le dispositif comprend en outre un système optique d'imagerie (9; 69) qui permet de réaliser l'image de la puce microstructurée (3; 33;43; 53; 63) sur le détecteur (10; 70).

Le système optique d'imagerie (9; 69) doit être suffisamment ouvert pour accepter tous les rayonnements issus de la puce microstructurée. La géométrie convexe des n protubérances dont est dotée la puce selon l'invention permet notamment de faire converger les faisceaux après réflexion du rayonnement incident sur ces dernières, rendant ainsi le système optique d'imagerie (9;69) beaucoup plus simple à réaliser.

De plus, le système optique d'imagerie (9; 69) est choisi pour que l'image de 2 protubérances ou de 1 protubérance et 1 cavité de la puce microstructurée correspondent à 2 positions différentes sur le détecteur (10; 70).

Enfin de manière avantageuse, le système optique présente un grandissement qui maximise le nombre de pixels utiles sur le détecteur (10; 70).

A titre d'exemple comme système optique d'imagerie (9; 69), on peut citer 2 lentilles plan-convexe montées en afocal.

Selon l'invention, on appelle face d'entrée la face par laquelle rentre un rayonnement incident dans la puce et face de sortie la face par laquelle sort le rayonnement réfléchi par la surface sensible.

Sur la figure 8, la source 7 émet un rayonnement incident, qui est collimaté à l'aide du système de collimation 8 et polarisé à l'aide du polariseur 6 avant d'arriver sur la face d'entrée 54 de la puce 53 (recouverte d'une couche métallique 52) sous une incidence donnée. Le rayonnement est dévié à son entrée dans la puce 53 et vient se réfléchir sur les protubérances 51.

Le système d'imagerie 9, situé après la face de sortie 57 de ladite puce 53, permet de collecter l'intensité des rayonnements réfléchis et de réaliser l'image des protubérances irradiées 51 sur le détecteur 10.

Le dispositif représenté sur la figure 9 comprend une puce 61 (recouverte d'une couche métallique 62) dont les faces latérales sont perpendiculaires à la base et dont les distances entre les surfaces séparant les protubérances 61 de la base sont différentes les unes des autres.

La figure 9 représente un mode de réalisation dans lequel le rayonnement incident émis par la source (non représentée sur la figure 9) qui arrive perpendiculairement sur la face d'entrée 64 (correspondant à la face latérale), n'est pas dévié lorsqu'il traverse la puce 63, et irradie l'ensemble des différentes protubérances 61.

Le système d'imagerie 69 et le détecteur 70 sont situés après la face de sortie 67, qui dans ce mode de réalisation correspond à la base de ladite puce 63, permettant ainsi de collecter l'intensité des rayonnements réfléchis et de réaliser l'image des protubérances 61.

Selon un mode de réalisation, le système optique d'imagerie (9; 69) peut être intégré directement dans la face de sortie (57; 67) de la puce (53; 63) sous la forme d'une matrice de microlentilles.

Selon un mode de réalisation préféré, le système de collimation 8 et éventuellement le polariseur 6 sont rendus solidaires de la face d'entrée (54; 64) et/ou le système optique d'imagerie (9;69) et le détecteur (10; 70) sont rendus solidaires de la face de sortie (57; 67).

### Procédé de mesure

Un autre objet de l'invention concerne un procédé de mesure par SPR qui comporte les étapes suivantes :
- détecter un état initial (i) en irradiant la surface sensible d'au moins une des n protubérances et m cavités par la face d'entrée de la puce microstructurée telle que définie selon une des revendications 1 à 12 à l'aide d'un faisceau incident monochromatique préalablement polarisé et éventuellement collimaté et (ii) en détectant simultanément l'intensité des rayonnements réfléchis par la surface sensible d'au moins une desdites n protubérances et m cavités, sortant par la face de sortie;
- mettre en contact au moins un fluide avec la surface sensible d'au moins une desdites n protubérances et m cavités;
- irradier la surface sensible d'au moins une desdites n protubérances et m cavités contenant ledit fluide par la face d'entrée de la puce microstructurée à l'aide d'un faisceau incident monochromatique préalablement polarisé, et éventuellement collimaté; et détecter simultanément l'intensité des rayonnements réfléchis par la surface sensible d'au moins une desdites n protubérances et m cavités, sortant par la face de sortie pour suivre en temps réel et en continu des modifications d'épaisseur optique dans au moins une desdites n protubérances et m cavités; avec n>0 et m≥0.

Un autre objet de l'invention concerne un procédé de mesure par SPR qui comporte les étapes suivantes :
- immobiliser des ligands sur la face supérieure recouverte d'une couche métallique d'une puce microstructurée telle que définie précédemment;
- détecter un état initial (i) en irradiant la surface sensible d'au moins une des n protubérances et m cavités par la face d'entrée de la puce microstructurée à l'aide d'un faisceau incident monochromatique préalablement polarisé, et éventuellement collimaté; et (ii) en détectant simultanément l'intensité des rayonnements réfléchis par la surface sensible d'au moins une desdites n protubérances et m cavités, sortant par la face de sortie;
- mettre en contact au moins un fluide avec la surface sensible d'au moins une desdites n protubérances et m cavités de ladite puce microstructurée;
- irradier la surface sensible d'au moins une desdites n protubérances et m cavités contenant ledit fluide par la face d'entrée de la puce microstructurée à l'aide d'un faisceau incident monochromatique préalablement polarisé, et éventuellement collimaté; et détecter simultanément l'intensité des rayonnements réfléchis par la surface sensible d'au moins une desdites n protubérances et m cavités, sortant par la face de sortie pour suivre en temps réel et en continu des modifications d'épaisseur optique dans au moins une desdites n protubérances et m cavités;
avec n>0 et m≥0.

Selon l'invention, l'immobilisation des ligands sur la surface supérieure peut être réalisée avec diverses techniques bien connues de l'homme de l'art comme par exemple l'immobilisation par liaison chimique covalente ou par électro-copolymérisation de pyrrole sur la surface métallique.

Par fluide on entend un gaz ou un liquide.

Selon un mode de réalisation préféré de l'invention, le fluide comprend au moins une espèce biologique.

Ces procédés de mesure sont adaptés pour mesurer, de manière non limitative, des variations de conformation de molécules immobilisées sur une surface, des interactions biomoléculaires, des indices optiques de fluides (gaz ou liquides), la qualité d'une surface (parallélisme, rugosité microscopique, qualité d'un dépôt de couches minces) ou encore la présence de nanobilles métalliques à proximité de la surface.

Ces procédés de mesure permettent également de mesurer des paramètres extérieurs tels que l'indice optique du milieu extérieur qui permet de remonter à la valeur de l'angle de réfraction limite.

Selon un mode de réalisation, le faisceau incident entre perpendiculairement par la face d'entrée 64 de la puce 63.

La détection de l'intensité des rayonnements réfléchis dans la plage angulaire Δθ (ou Δθ') par le détecteur (10; 70) permet de générer toute ou une partie de la courbe plasmon dont les principales zones d'intérêt sont les suivantes :
- le minimum de sensibilité plasmon;
- la plage angulaire dans laquelle la sensibilité est la plus forte (c'est-à-dire là où la dérivée du plasmon est la plus importante) appelée également « flanc du plasmon »;
- la zone aux alentours de l'angle de réfraction limite (où on passe d'un régime de réfraction à un régime de réflexion totale).

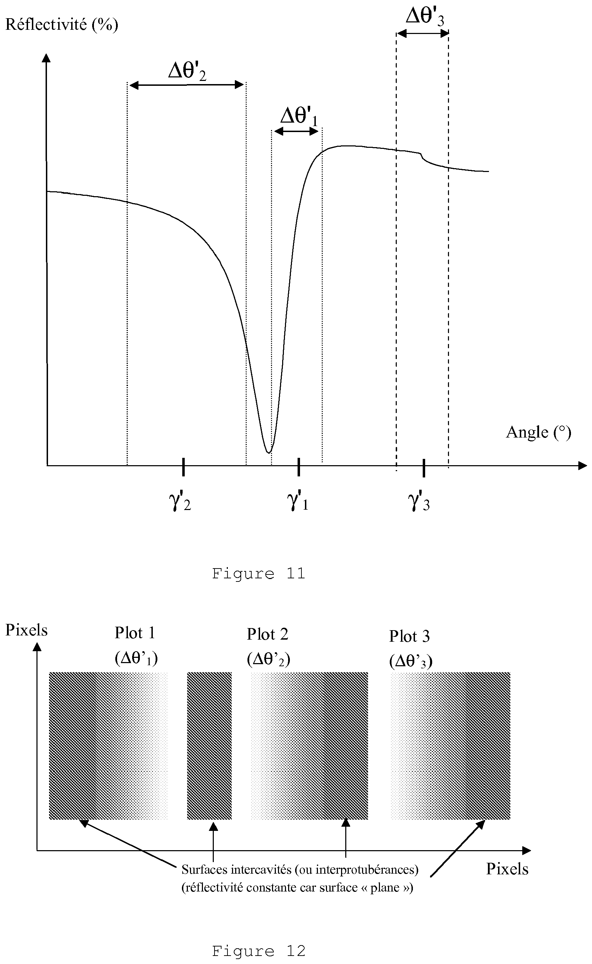

Les figures 10 et 11 représentent une courbe plasmon de 3 zones choisies parmi n protubérances et m cavités avec n+m=3 présentant des plages angulaires d'études différentes (1^{ère} protubérance ayant une plage angulaire Δθ₁ pour la figure 10 et Δθ₁' pour la figure 11, 2^{ème} cavité et/ou protubérance ayant une plage angulaire Δθ₂ pour la figure 10 et Δθ₂' pour la figure 11 et 3^{ème} cavité et/ou protubérance ayant une plage angulaire Δθ₃ pour la figure 10 et Δθ₃' pour la figure 11).

La figure 10 représente les plages angulaires de 3 zones choisies parmi n protubérances et m cavités avec n+m=3 lorsque β=0 (sans tilt) alors que la figure 11 représente les plages angulaires de 3 choisies parmi n protubérances et m cavités avec n+m=3 lorsque β≠0 (avec tilt) .

Dans la figure 10, c'est-à-dire lorsque β=0 (sans tilt), les 3 plages angulaires étudiées sont centrées sur l'angle θ. La largeur de chacune des zones est définie par l'angle au sommet 2α et le rayon de courbure R de chacune des protubérances ou protubérances et cavités. Le choix de l'angle θ est donc primordial pour observer au mieux toutes les zones d'intérêt. Il est ainsi possible, en fonction du rayon de courbure de la zone étudiée, d'explorer une plage angulaire de la courbe plasmon plus ou moins grande. Dans le cas où les zones ne présentent pas de tilt β (c'est le cas pour la figure 10) cette plage angulaire sera toujours centrée sur la même valeur θ.

Dans la figure 11, c'est-à-dire lorsque β≠0 (avec tilt), les trois plages angulaires Δθ'₁, Δθ'₂ et Δθ'₃ sont centrées respectivement sur les angles γ'₁, γ'₂ et γ'₃, lesdits angles γ'₁, γ'₂ et γ'₃ étant chacun définis par des angles β différents. Dans ce cas de figure (avec tilt), les plages angulaires ne sont pas centrées sur la même valeur. Il est ainsi possible d'explorer une plage angulaire variable de la courbe plasmon tout en ayant un faisceau incident à un angle unique, permettant ainsi de ne pas avoir besoin des pièces mobiles habituellement utilisées pour effectuer une rotation angulaire du faisceau incident.

Selon un autre mode de réalisation, le procédé de mesure comprend en outre une étape dans laquelle une image des n protubérances et des m cavités de la puce est réalisée.

La figure 12 représente l'image des 3 zones choisies parmi n protubérances et m cavités avec n+m=3 (courbe plasmon de la figure 11) sur un détecteur matriciel dans laquelle la bande noire entre les bandes lumineuses (en dégradé de gris) représente les surfaces inter-cavités et/ou inter-protubérances qui ont une réflectivité constante car ce sont des surfaces planes.

Selon l'invention, on peut ainsi, connaissant l'angle incident θ constant et fixé par l'architecture du système optique (puisqu'aucune pièce mécanique n'est en mouvement), choisir la plage angulaire Δθ qui sera éventuellement imagée sur le détecteur (en choisissant le rayon de courbure R et l'angle α, ou le rayon de courbure R et le diamètre d, car α et d se déduisent l'un de l'autre grâce au rayon de courbure R) et l'angle moyen γ'_{moyen} (avec le choix du tilt β) de la microstructure en fonction des différentes espèces à analyser avec γ'_{moy} =θ+2β. Ce choix peut être fait pour chacune des n protubérances et des m cavités, ce qui permet de s'adapter à plusieurs types d'espèces immobilisées sur la même puce.

De même, le choix peut être fait en fonction du type d'espèces qui vont venir interagir avec les espèces préalablement immobilisées sur la puce : on peut ainsi « adapter » chacune des protubérances ou protubérances et cavités à l'espèce recherchée.

Ainsi, un autre objet de l'invention concerne l'utilisation du dispositif selon l'invention pour la mesure d'interactions biomoléculaires.

L'invention est illustrée à l'aide des exemples qui sont donnés à titre d'illustration uniquement et ne sont pas limitatifs.

### Exemple 1 : Puce à protéines - suivi et correction de l'indice du milieu extérieur en temps réel dans des cinétiques d'interactions

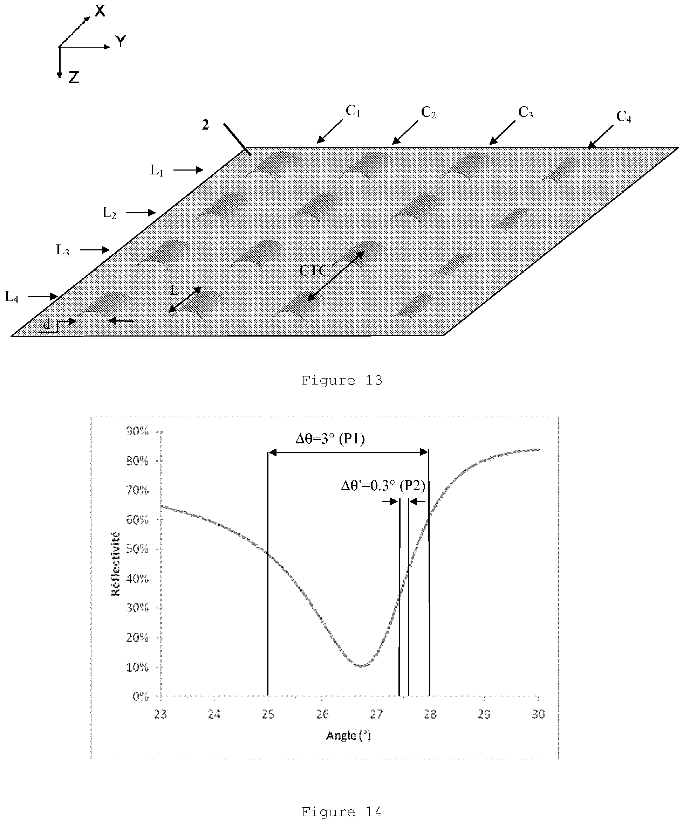

Une puce microstructurée réalisée en polycarbonate (PC) comprenant 16 protubérances est représentée sur la figure 13.

Une couche d'or d'une épaisseur de 48nm a été déposée par pulvérisation cathodique sur la face supérieure de ladite puce afin d'obtenir un effet plasmon.

En polarisation TM, le minimum de réflectivité est obtenu aux alentours de l'angle θ =28°.

Les 16 protubérances sont réparties suivant une matrice régulière de 4x4 surfaces semi-cylindriques de diamètre d=500pm et de longueur L=500pm, chacune espacée d'une distance centre-à-centre (CTC) de 1mm.

Quatre espèces différentes ont été immobilisées de manière covalente grâce à une électro-polymérisation d'un film de polypyrrole fonctionnalisé avec les ligands d'intérêt.

Ainsi, les espèces suivantes présentes dans les protubérances sont :
- sur la ligne L₁ : anticorps monoclonaux anti-souris
- sur la ligne L₂ : anticorps monoclonaux dirigés contre l'hormone gonadotrophine chorionique humaine (hCG, impliquée en tant que biomarqueur dans plusieurs pathologies cancéreuses)
- sur la ligne L_{3 :} anticorps monoclonaux dirigés contre la bactérie *Listeria monocytogenes*
- sur la ligne L₄ :de la BSA (« bovin serum albumine »).

La puce est irradiée avec un rayonnement incident polarisé, collimaté et monochromatique et l'intensité des rayonnements réfléchis par les protubérances est détectée par un détecteur de type CMOS.

Le rayon de courbure des surfaces semi-cylindriques des protubérances des colonnes C₁, C₂ et C₃, est de 9,5mm (équivalent à une zone angulaire d'étude Δθₐ sur la courbe plasmon d'environ 3°) et les protubérances de la colonne C₄ présentent un rayon de courbure de 1,9mm (équivalent à une zone angulaire d'étude Δθ_{b} d'environ 15°). Les plages angulaires Δθₐ et Δθ_{b} correspondent respectivement aux plages angulaires Δθ₁ et Δθ₃ sur la figure 10.

Un liquide d'un indice optique inconnu contenant des protéines hCG est mis en contact avec la puce dans les protubérances. Une variation caractéristique du signal est observée en fonction du temps sur les protubérances de la ligne L₂ mais pas sur les autres car une interaction spécifique a lieu sur les anticorps monoclonaux anti hCG et pas sur les autres protéines immobilisées.

De plus, puisque la protubérance située en (L₂,C₄) présente un rayon de courbure tel que toute la courbe plasmon peut être visualisée sur le détecteur, la valeur angulaire de l'angle de réfraction limite peut être aisément déterminé et il est ainsi possible de déduire l'indice du milieu liquide inconnu.

Ainsi, connaissant cet indice, il est possible de déterminer avec précision la variation du signal sur les protubérances situées en (L₂,C₁), (L₂,C₂) et (L₂,C₃), en décorrélant la variation de signal liée au milieu extérieur de la variation de signal liée à l'accrochage de protéines hCG sur les anticorps monoclonaux anti-hCG.

### Exemple 2 : puce pour l'étude de bactéries Escherichia coli émettrices de shigatoxines

Dans cet exemple, la présence de bactéries *Escherichia coli* émettrices de shigatoxines (STEC) dans un échantillon issu de l'industrie agro-alimentaire a été étudiée.

Des exemples de bactéries de cette catégorie sont les souches O157:H7, O26:H11 ou encore O103:H2. Les bactéries de ce type et les shigatoxines qu'elles produisent sont à l'origine de troubles intestinaux sévères, pour lesquels le pronostic vital peut être engagé. La taille et le poids moléculaire de ces shigatoxines (quelques nanomètres de diamètre et un poids moléculaire d'environ 68kDa) est très différent de celui d'une bactérie *Escherichia coli* (environ 10 millions de fois plus lourde). Par conséquent, les signaux plasmon pour l'accrochage de ces 2 types de familles sont très différents. Avec des appareils SPR classiques, on ne peut donc pas étudier à la fois ces bactéries et ces toxines lors d'une même expérience et en temps réel. En effet, dans le cas d'accrochage de bactéries, la courbe va se décaler angulairement de près de 0,1° tandis qu'elle ne va bouger que de moins de 0,01° dans le cas de toxines (ce qui n'est pas facilement détectable par des méthodes SPR classiques).

Cet exemple démontre qu'en utilisant une puce microstructurée selon l'invention qui présente une architecture particulière, il est possible d'étudier ces 2 espèces biologiques de tailles différentes au cours d'une seule expérience.

Une puce réalisée en polycarbonate comprenant deux protubérances semi-cylindriques, de diamètre 600pm et de longueur 800pm est réalisée.

La face supérieure de ladite puce est recouverte par une couche de chrome d'une épaisseur de 2nm et d'une couche d'or d'une épaisseur de 48nm toutes les deux déposées par pulvérisation cathodique afin d'obtenir un effet plasmon.

La première protubérance présente un tilt nul (β=0) et la seconde protubérance présente un tilt de valeur β=0,5°.

La puce est irradiée avec un rayonnement incident polarisé, collimaté et monochromatique et l'intensité des rayonnements réfléchis par la surface sensible des protubérances est détectée par un détecteur de type CCD.

L'angle moyen d'incidence du système optique est fixé mécaniquement par l'architecture du système et est égal à 26,5°.

Le rayon de courbure des deux protubérances est également différent : 11,5mm pour la première protubérance (correspondant à une plage angulaire d'étude Δθ de 3°) et 100mm pour la deuxième (ce qui correspond à une plage angulaire d'analyse Δθ' de 0,3°) (figure 14).

Des anticorps monoclonaux dirigés contre la bactérie O157:H7 sont immobilisés de manière covalente et de manière uniforme sur la première protubérance et des anticorps monoclonaux dirigés spécifiquement contre les shigatoxines que la bactérie sécrète sont immobilisés sur la deuxième protubérance.

Les deux courbes plasmons générées à partir de ces 2 protubérances présentent le même angle de résonance plasmon (environ 26,7°), avant réaction.

Lorsqu'un mélange contenant une grande quantité de bactéries O157:H7 est mis en contact avec la surface sensible de la puce (autrement dit sur les protubérances), certaines d'entres elles interagissent spécifiquement avec les anticorps de la première protubérance. Comme les bactéries sont des molécules bien détectées par la SPR (du fait de leur poids important), la courbe plasmon se décale suffisamment pour que ce décalage soit très bien perçu par le détecteur imageant la première protubérance. Par ailleurs, cette bactérie sécrète également des shigatoxines au cours de la même expérience.

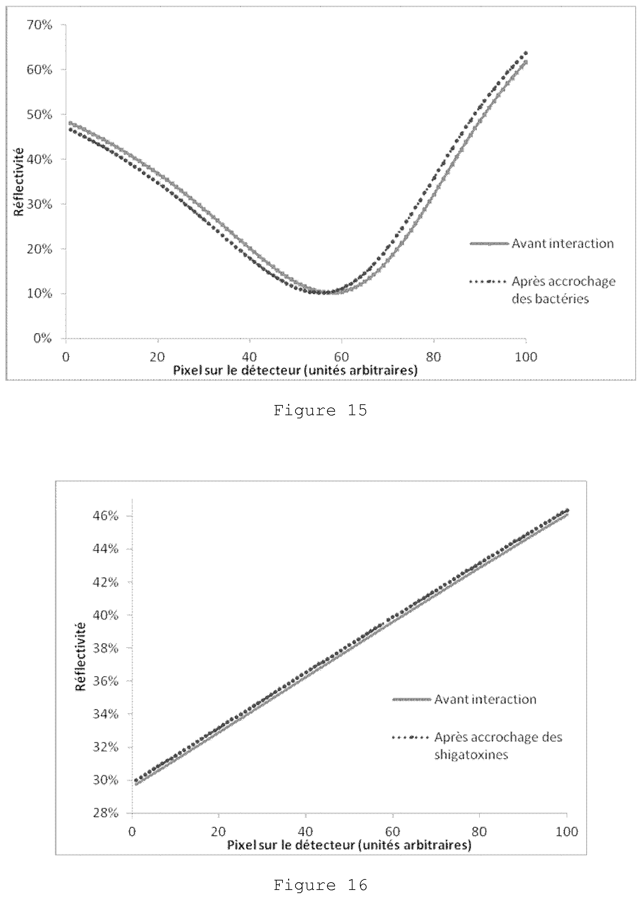

La deuxième protubérance présente un léger tilt (β=0,5°) et une largeur angulaire étudiée beaucoup plus faible (Δθ'=0,3°). Ainsi, la sensibilité de mesure de la seconde protubérance est plus importante et il est possible de détecter des shigatoxines venant se fixer sur les anticorps de la deuxième protubérance. Les figures 15 et 16 représentent les signaux obtenus sur le détecteur pour les deux protubérances avant et après interaction des anticorps immobilisés sur chacune des protubérances avec respectivement les bactéries et les toxines.

Ainsi, cet exemple démontre que la puce selon l'invention permet d'adapter la dynamique et la sensibilité de mesure aux espèces à mesurer au cours d'une seule expérience.

## Revendications

1. Puce microstructurée (3; 33; 43; 53; 63) pour analyse par résonance des plasmons de surface (SPR) se présentant sous la forme d'un solide constitué d'une base (5; 77), d'une face supérieure (4; 44) dont au moins une partie est recouverte d'une couche métallique (2; 22; 42; 52; 62), et d'au moins une face latérale (55; 66), dans laquelle :
- ladite face supérieure est dotée de zones (1; 11; 41; 51; 61) d'une taille allant de 1 µm à 1000 µm destinées à recevoir des espèces à analyser choisies parmi n protubérances et m cavités; et
- lorsque n+m≥ 2, lesdites zones sont séparées les unes des autres par des surfaces planes, lesdites surfaces planes étant parallèles à un plan (XY),
avec n allant de 1 à j, m allant de 0 à i; j et i étant des entiers ;
- lesdites zones (1; 11; 41; 51; 61) présentent une surface courbe de rayon de courbure R moyen situé dans un plan orthogonal au plan (XY) ; et
- le rayon de courbure R moyen de la surface courbe est compris entre 0,1 et 600mm, **caractérisée en ce que** au moins une des n protubérances et m cavités (1; 11; 41; 51; 61) présente un rayon de courbure différent des autres.

2. Puce microstructurée (3; 33; 43; 53; 63) selon la revendication 1, **caractérisée par le fait qu'**au moins une zone choisie parmi les n protubérances et les m cavités est recouverte par la couche métallique.

3. Puce microstructurée (3; 33; 43; 53; 63) selon la revendication 1 ou 2, **caractérisée par le fait que** m=0.

4. Puce microstructurée (3; 33; 43; 53; 63) selon la revendication 1 ou 2, **caractérisée par le fait que** m>0.

5. Puce microstructurée (3; 33; 43; 53; 63) selon l'une des revendications 1 à 4, **caractérisée en ce que** la base (5; 77) est une surface plane.

6. Puce microstructurée (3; 33; 43; 53; 63) selon l'une des revendications 1 à 5, **caractérisée en ce que** la face supérieure (4;44) est parallèle à la base (5; 77).

7. Puce microstructurée (3; 33; 43; 53; 63) selon l'une des revendications 1 à 5, **caractérisée en ce qu'**au moins une face latérale (55; 66) est plane.

8. Puce microstructurée (3; 33; 43; 53; 63) selon la revendication 6, **caractérisée en ce qu'**au moins une face latérale (55; 66) est plane.

9. Puce microstructurée (3; 33; 43; 53; 63) selon la revendication 7, **caractérisée en ce que** au moins une face latérale (55; 66) est perpendiculaire à la base (5; 77) ou à la face supérieure(4;44).

10. Puce microstructurée (3; 33; 43; 53; 63) selon la revendication 8, **caractérisée en ce que** au moins une face latérale (55; 66) est perpendiculaire à la base (5; 77) et à la face supérieure(4;44).

11. Puce microstructurée (3; 33; 43; 53; 63) selon l'une des revendications 1 à 10, **caractérisée en ce qu'**au moins une des n protubérances et m cavités (1; 11; 41; 51; 61) présente un basculement d'un angle β, de préférence tel que 0°< β≤80°.

12. Puce microstructurée (3; 33; 43; 53; 63) selon l'une des revendications 1 à 11, **caractérisée en ce qu'**au moins une des distances séparant les surfaces planes entre les n protubérances et m cavités (1; 11; 41; 51; 61) de la base (5; 77) est différente des autres.

13. Dispositif d'analyse par SPR comprenant:
- une source lumineuse (7) destinée à générer un faisceau incident;
- éventuellement un système optique de collimation (8);
- un système polarisant (6);
- une puce microstructurée (3; 33; 43; 53; 63) telle que définie dans l'une des revendications 1 à 12, disposée dans le trajet optique dudit faisceau incident;
- éventuellement un système optique d'imagerie (9; 69);
- un détecteur (10; 70).

14. Dispositif selon la revendication 13, **caractérisé en ce que** le système de collimation (8) et éventuellement le polariseur (6) sont rendus solidaire de la face d'entrée (54; 64) et/ou le système optique d'imagerie (9; 69) et le détecteur (10; 70) sont rendus solidaire de la face de sortie (57; 67).

15. Procédé de mesure par SPR qui comporte les étapes suivantes :
- détecter un état initial (i) en irradiant la surface sensible d'au moins une des n protubérances et m cavités par la face d'entrée de la puce microstructurée telle que définie selon l'une des revendications 1 à 12 à l'aide d'un faisceau incident monochromatique préalablement polarisé et éventuellement collimaté et (ii) en détectant simultanément l'intensité des rayonnements réfléchis par la surface sensible d'au moins une desdites n protubérances et m cavités, sortant par la face de sortie;
- mettre en contact au moins un fluide avec la surface sensible d'au moins une desdites n protubérances et m cavités;
- irradier la surface sensible d'au moins une desdites n protubérances et m cavités contenant ledit fluide par la face d'entrée de la puce microstructurée à l'aide d'un faisceau incident monochromatique préalablement polarisé et éventuellement collimaté et détecter simultanément l'intensité des rayonnements réfléchis par la surface sensible d'au moins une desdites n protubérances et m cavités, sortant par la face de sortie pour suivre en temps réel et en continu des modifications d'épaisseur optique dans au moins une desdites n protubérances et m cavités;
avec n>0 et m≥0.

16. Procédé de mesure par SPR selon la revendication 15, **caractérisé en ce qu'**il comprend préalablement une étape consistant à immobiliser des ligands sur la face supérieure recouverte d'une couche métallique d'une puce microstructurée telle que définie selon l'une des revendications 1 à 12.

17. Procédé selon l'une des revendications 15 ou 16, **caractérisé en ce qu'**il comporte en outre une étape finale dans laquelle une image des n protubérances et m cavités (1; 11; 41; 51; 61) de la puce est réalisée.

18. Utilisation du dispositif tel que défini selon l'une des revendications 13 ou 14 pour mesurer des interactions biomoléculaires.

## Patentansprüche

1. Mikrostrukturierter Chip (3; 33; 43; 53; 63) zur Oberflächenplasmonresonanzanalyse (SPR-Analyse), welcher in der Form eines Festkörpers vorliegt, der aus einer Grundfläche (5; 77), einer Oberseite (4; 44), von der wenigstens ein Teil mit einer metallischen Schicht (2; 22; 42; 52; 62) bedeckt ist, und wenigstens einer Seitenfläche (55; 66) besteht, wobei:
- die Oberseite mit Bereichen (1; 11; 41; 51; 61) mit einer Größe von 1 µm bis 1000 µm ausgestattet ist, die dazu bestimmt sind, zu analysierende Spezies aufzunehmen, die aus n Erhebungen und m Vertiefungen ausgewählt sind; und
- wenn n+m ≥ 2 ist, die Bereiche durch ebene Flächen voneinander getrennt sind, wobei die ebenen Flächen parallel zu einer Ebene (XY) sind, mit n von 1 bis j, m von 0 bis i; wobei j und i ganze Zahlen sind;
- die Bereiche (1; 11; 41; 51; 61) eine gekrümmte Oberfläche mit einem mittleren Krümmungsradius R aufweisen, der sich in einer zur Ebene (XY) orthogonalen Ebene befindet; und
- der mittlere Krümmungsradius R der gekrümmten Oberfläche zwischen 0,1 und 600 mm liegt,
**dadurch gekennzeichnet, dass** wenigstens eine der n Erhebungen und m Vertiefungen (1; 11; 41; 51; 61) einen Krümmungsradius aufweist, der von den anderen verschieden ist.

2. Mikrostrukturierter Chip (3; 33; 43; 53; 63) nach Anspruch 1, **dadurch gekennzeichnet, dass** wenigstens ein aus den n Erhebungen und m Vertiefungen ausgewählter Bereich von der metallischen Schicht bedeckt ist.

3. Mikrostrukturierter Chip (3; 33; 43; 53; 63) nach Anspruch 1 oder 2, **dadurch gekennzeichnet, dass** m = 0 ist.

4. Mikrostrukturierter Chip (3; 33; 43; 53; 63) nach Anspruch 1 oder 2, **dadurch gekennzeichnet, dass** m > 0 ist.

5. Mikrostrukturierter Chip (3; 33; 43; 53; 63) nach einem der Ansprüche 1 bis 4, **dadurch gekennzeichnet, dass** die Grundfläche (5; 77) eine ebene Fläche ist.

6. Mikrostrukturierter Chip (3; 33; 43; 53; 63) nach einem der Ansprüche 1 bis 5, **dadurch gekennzeichnet, dass** die Oberseite (4; 44) parallel zur Grundfläche (5; 77) ist.

7. Mikrostrukturierter Chip (3; 33; 43; 53; 63) nach einem der Ansprüche 1 bis 5, **dadurch gekennzeichnet, dass** wenigstens eine Seitenfläche (55; 66) eben ist.

8. Mikrostrukturierter Chip (3; 33; 43; 53; 63) nach Anspruch 6, **dadurch gekennzeichnet, dass** wenigstens eine Seitenfläche (55; 66) eben ist.

9. Mikrostrukturierter Chip (3; 33; 43; 53; 63) nach Anspruch 7, **dadurch gekennzeichnet, dass** wenigstens eine Seitenfläche (55; 66) senkrecht zur Grundfläche (5; 77) oder zur Oberseite (4; 44) ist.

10. Mikrostrukturierter Chip (3; 33; 43; 53; 63) nach Anspruch 8, **dadurch gekennzeichnet, dass** wenigstens eine Seitenfläche (55; 66) senkrecht zur Grundfläche (5; 77) und zur Oberseite (4; 44) ist.

11. Mikrostrukturierter Chip (3; 33; 43; 53; 63) nach einem der Ansprüche 1 bis 10, **dadurch gekennzeichnet, dass** wenigstens eine der n Erhebungen und m Vertiefungen (1; 11; 41; 51; 61) eine Schwenkung um einen Winkel β aufweist, vorzugsweise derart, dass 0° < β ≤ 80° ist.

12. Mikrostrukturierter Chip (3; 33; 43; 53; 63) nach einem der Ansprüche 1 bis 11, **dadurch gekennzeichnet, dass** wenigstens einer der Abstände, der die ebenen Flächen zwischen den n Erhebungen und m Vertiefungen (1; 11; 41; 51; 61) von der Grundfläche (5; 77) trennt, von den anderen verschieden ist.

13. Vorrichtung zur SPR-Analyse, welche umfasst:
- eine Lichtquelle (7), die dazu bestimmt ist, einen einfallenden Strahl zu erzeugen;
- eventuell ein optisches Kollimationssystem (8);
- ein Polarisationssystem (6);
- einen mikrostrukturierten Chip (3; 33; 43; 53; 63), wie in einem der Ansprüche 1 bis 12 definiert, der im Strahlengang des einfallenden Strahls angeordnet ist;
- eventuell ein optisches Bildgebungssystem (9; 69) ;
- einen Detektor (10; 70).

14. Vorrichtung nach Anspruch 13, **dadurch gekennzeichnet, dass** das Kollimationssystem (8) und eventuell der Polarisator (6) mit der Eintrittsseite (54; 64) fest verbunden sind und/oder das optische Bildgebungssystem (9; 69) und der Detektor (10; 70) mit der Austrittsseite (57; 67) fest verbunden sind.

15. SPR-Messverfahren, welches die folgenden Schritte umfasst:
- Erfassen eines Anfangszustands (i) durch Bestrahlen der empfindlichen Oberfläche wenigstens einer der n Erhebungen und m Vertiefungen über die Eintrittsseite des mikrostrukturierten Chips, wie er in einem der Ansprüche 1 bis 12 definiert ist, mithilfe eines einfallenden monochromatischen Strahls, der zuvor polarisiert und eventuell kollimiert wurde, und (ii) durch gleichzeitiges Erfassen der Intensität der von der empfindlichen Oberfläche wenigstens einer der n Erhebungen und m Vertiefungen reflektierten Strahlungen, die über die Austrittsseite austreten;
- Inkontaktbringen wenigstens eines Fluids mit der empfindlichen Oberfläche wenigstens einer der n Erhebungen und m Vertiefungen;
- Bestrahlen der empfindlichen Oberfläche wenigstens einer der n Erhebungen und m Vertiefungen, die das Fluid enthalten, über die Eintrittsseite des mikrostrukturierten Chips mithilfe eines einfallenden monochromatischen Strahls, der zuvor polarisiert und eventuell kollimiert wurde, und gleichzeitiges Erfassen der Intensität der von der empfindlichen Oberfläche wenigstens einer der n Erhebungen und m Vertiefungen reflektierten Strahlungen, die über die Austrittsseite austreten, um in Echtzeit und kontinuierlich Änderungen der optischen Dicke in wenigstens einer der n Erhebungen und m Vertiefungen zu verfolgen;
mit n > 0 und m ≥ 0.

16. SPR-Messverfahren nach Anspruch 15, **dadurch gekennzeichnet, dass** es einen vorausgehenden Schritt umfasst, der darin besteht, Liganden auf der mit einer metallischen Schicht bedeckten Oberseite eines mikrostrukturierten Chips, wie er in einem der Ansprüche 1 bis 12 definiert ist, zu immobilisieren.

17. Verfahren nach einem der Ansprüche 15 oder 16, **dadurch gekennzeichnet, dass** es außerdem einen abschließenden Schritt umfasst, in welchem ein Bild der n Erhebungen und m Vertiefungen (1; 11; 41; 51; 61) des Chips hergestellt wird.

18. Verwendung der Vorrichtung, wie sie in einem der Ansprüche 13 oder 14 definiert ist, um biomolekulare Interaktionen zu messen.

## Claims

1. Microstructured chip (3; 33; 43; 53; 63) for surface plasmon resonance (SPR) analysis which is in the form of a solid consisting of a base (5; 77), of an upper face (4; 44), at least one part of which is covered with a metal layer (2; 22; 42; 52; 62), and of at least one side face (55; 66), in which:
- said upper face is provided with zones (1; 11; 41; 51; 61) having a size ranging from 1 µm to 1000 µm intended to receive species to be analysed, chosen from n protuberances and m cavities; and
- when n + m ≥ 2, said zones are separated from one another by planar surfaces, said planar surfaces being parallel to a plane (XY),
with n ranging from 1 to j, m ranging from 0 to i; j and i being integers;
- said zones (1; 11; 41; 51; 61) having a curved surface with an average radius of curvature R located in a plane orthogonal to the plane (XY); and
- the average radius of curvature R of the curved surface is between 0.1 and 600 mm,
**characterized in that** at least one of the n protuberances and m cavities (1; 11; 41; 51; 61) has a radius of curvature different from the others.

2. Microstructured chip (3; 33; 43; 53; 63) according to Claim 1, **characterized in that** at least one zone chosen from the n protuberances and the m cavities is covered with the metal layer.

3. Microstructured chip (3; 33; 43; 53; 63) according to Claim 1 or 2, **characterized in that** m = 0.

4. Microstructured chip (3; 33; 43; 53; 63) according to Claim 1 or 2, **characterized in that** m > 0.

5. Microstructured chip (3; 33; 43; 53; 63) according to one of Claims 1 to 4, **characterized in that** the base (5; 77) is a planar surface.

6. Microstructured chip (3; 33; 43; 53; 63) according to one of Claims 1 to 5, **characterized in that** the upper face (4; 44) is parallel to the base (5; 77) .

7. Microstructured chip (3; 33; 43; 53; 63) according to one of Claims 1 to 5, **characterized in that** at least one side face (55; 66) is planar.

8. Microstructured chip (3; 33; 43; 53; 63) according to Claim 6, **characterized in that** at least one side face (55; 66) is planar.

9. Microstructured chip (3; 33; 43; 53; 63) according to Claim 7, **characterized in that** at least one side face (55; 66) is perpendicular to the base (5; 77) or to the upper face (4; 44).

10. Microstructured chip (3; 33; 43; 53; 63) according to Claim 8, **characterized in that** at least one side face (55; 66) is perpendicular to the base (5; 77) and to the upper face (4; 44).

11. Microstructured chip (3; 33; 43; 53; 63) according to one of Claims 1 to 10, **characterized in that** at least one of the n protuberances and m cavities (1; 11; 41; 51; 61) has a tilt of an angle β, preferably such that 0° < β ≤ 80°.

12. Microstructured chip (3; 33; 43; 53; 63) according to one of Claims 1 to 11, **characterized in that** at least one of the distances separating the planar surfaces between the n protuberances and m cavities (1; 11; 41; 51; 61) and the base (5; 77) is different from the others.

13. Device for SPR analysis comprising:
- a light source (7) intended to generate an incident beam;
- optionally an optical collimation system (8);
- a polarizing system (6);
- a microstructured chip (3; 33; 43; 53; 63) as defined in one of Claims 1 to 12, placed in the optical path of said incident beam;
- optionally an optical imaging system (9; 69);
- a detector (10; 70).

14. The device according to Claim 13, **characterized in that** the collimation system (8) and optionally the polarizer (6) are secured to the entry face (54; 64) and/or the optical imaging system (9; 69) and the detector (10; 70) are secured to the exit face (57; 67).

15. Method of SPR measurement which comprises the following steps:
- detecting an initial state (i) by irradiating the sensitive surface of at least one of the n protuberances and m cavities via the entry face of the microstructured chip as defined in one of Claims 1 to 12 by means of a previously polarized and optionally collimated monochromatic incident beam; and (ii) by simultaneously detecting the intensity of the radiations reflected by the sensitive surface of at least one of said n protuberances and m cavities, exiting via the exit face;
- bringing at least one fluid into contact with the sensitive surface of at least one of said n protuberances and m cavities;
- irradiating the sensitive surface of at least one of said n protuberances and m cavities containing said fluid, via the entry face of the microstructured chip, by means of a previously polarized and optionally collimated monochromatic incident beam; and simultaneously detecting the intensity of the radiations reflected by the sensitive surface of at least one of said n protuberances and m cavities, exiting via the exit face, so as to continuously monitor, in real time, optical thickness modifications in at least one of said n protuberances and m cavities;
where n > 0 and m ≥ 0.

16. Method of SPR measurement according to Claim 15, **characterized in that** it comprises beforehand a step consisting in immobilizing ligands on the upper face, covered with a metal layer, of a microstructured chip as defined in one of Claims 1 to 12.

17. Method according to either of Claims 15 and 16, **characterized in that** it also comprises a final step in which an image of the n protuberances and m cavities (1; 11; 41; 51; 61) of the chip is produced.

18. Use of the device as defined in either of Claims 13 and 14, for measuring biomolecular interactions.
